# EUROPEAN PATENT SPECIFICATION

(11) **EP 1 053 739 B1**
(45) Date of publication and mention of the grant of the patent: **02.11.2005**
(21) Application number: 00110201.1
(22) Date of filing: 17.05.2000
(51) Int. Cl.: A61K 6/097, A61K 6/033, A61L 27/12, A61L 27/20

(54) **Method of manufacturing an osteoconductive substance**
Verfahren zur Herstellung eines osteokonduktiven Materials
Procédé de fabrication d'une substance ostéoconductrice

(30) Priority: 18.05.1999 JP 13669099
(43) Date of publication of application: 22.11.2000
(73) Proprietor: Matsumoto Dental University, Nagano 399-0781 (JP)
(72) Inventor: Ito, Michio, Shiojiri, Nagano 399-0742 (JP)
(74) Representative: Vossius & Partner

(56) References cited:
- EP-A- 0 329 098
- EP-A- 0 555 807
- EP-A- 0 754 466
- WO-A-97/14376

## Description

This invention relates to a method of manufacturing an osteoconduction substance for use mainly in dental, orthopedic, and plastic surgery fields to accelerate creation of new bone.

Generally, the osteoconduction substance is implanted in the vicinity or in the interior of an existing bone. In this state, the osteoconduction substance can create a new bone connected to the existing bone.

A conventional osteoconduction substance of the type described is known which comprises a hardenable composition containing chitosan. The hardenable composition is manufactured by a mixture of an acidic aqueous solution of chitosan, particulate hydoroxyapatite, zinc oxide and/or magnesium oxide. The conventional osteoconduction substance exhibits an excellent affinity to a tooth and a bone and is hardended within a neutral range. Such a hardenable composition is disclosed in Japanese Patent Prepublication (JP-A) No. H01-208347 (208347/1989).

However, the conventional osteoconduction substance is disadvantageous in that a long period of time is required until creation of the new bone from implantation of the osteoconduction substance.

It is therefore an object of the present invention to provide a method of manufacturing an osteoconduction substance which is capable of accelerating creation of a new bone.

Other objects of the present invention will become clear as the description proceeds.

According to the present invention, there is provided a method of manufacturing an osteoconduction substance. The method comprises the steps of dissolving a chitosan by an acid aqueous solution to produce a chitosan sol, mixing an apatite powder in said chitosan sol to produce a mixture, drying said mixture into a dried substance, and adsorbing Ringer's solution in said dried substance to produce an elastic body as said osteoconduction substance.

### Brief Description of the Drawing:

Fig. is a flow chart for describing a previous method of manufacturing an osteoconduction substance; and

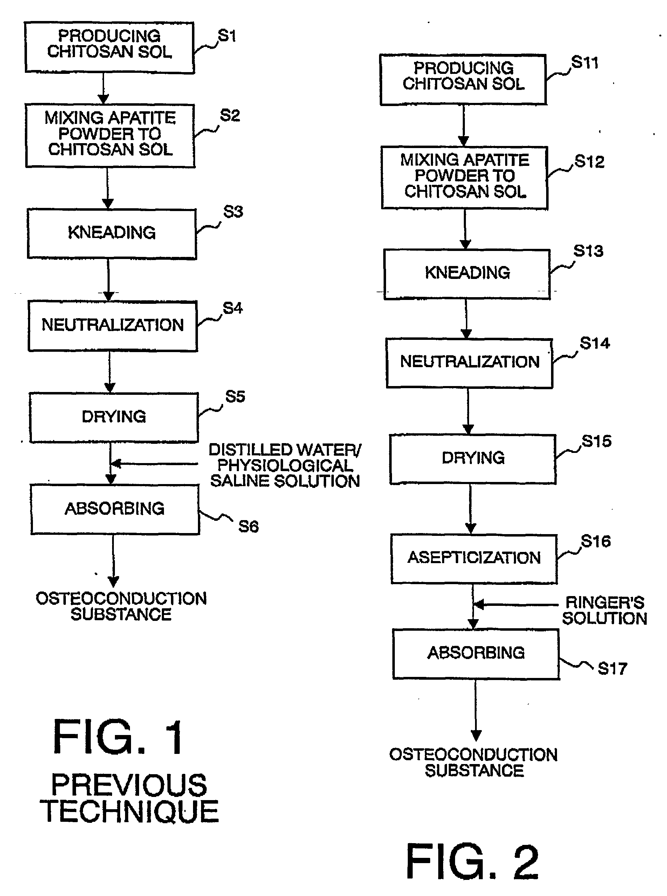

Fig. 2 is a flow chart for describing a method of manufacturing an osteoconduction substance according to an embodiment of the present invention.

### Description of the Preferred Embodiment:

Referring to Fig. 1, description will be made at first as regards a method of manufacturing an osteoconduction substance in a previous technique for a better understanding of the present invention.

In the manner which will presently be described, the method in the previous technique comprises a first step S1, a second step S2, a third step S3, a fourth step S4, a fifth step S5, and a sixth step S6.

At the first step S1, a chitosan is prepared. An acid aqueous solution is added to the chitosan to dissolve the chitosan. As a result of dissolving the chitosan, a chitosan sol is produced. At the second step S2, an apatite powder is added to or mixed with the chitosan sol. At the third step S3, a mixture of the apatite powder and the chitosan sol is kneaded into a kneaded mixture. At the fourth step S4, the kneaded mixture is neutralized by the aqueous solution including the compound. At fifth step S5, the kneaded mixture is dried into a dried substance. At sixth step S6, the dried substance is adsorbed with the distilled water or physiological saline solution. Thus, the dried substance is processed into the osteoconduction substance that has elastic strength like rubber.

The osteoconduction substance is cut into a plurality of pieces each of which may have an optimal size in correspondence to a diseased part. For example, the piece may be inserted between a jaw bone and a gingiva surrounding the jaw bone after operation for periodontitis in order reconstruct the jaw bone and to prevent recurrence of periodontitis.

With this osteoconduction substance, no migration and no loss of the apatite powder take place. This is because chitosan sol is transformed into gel to thereby fixedly confine the apatite. Before chitosan is completely absorbed into an organism, osteoid tissue is produced and substituted for a new bone.

However, the osteoconduction substance is advantageous in that a long period of time is not required until creation of the new bone from implantation of the osteoconduction substance.

Referring to Fig. 2, the description will be made as regards a method of manufacturing an osteoconduction substance according to an embodiment of this invention.

In the manner which will presently be described, the method comprises a first step S11, a second step S12, a third step S13, a fourth step S14, a fifth step S15, a sixth step S16, and a seventh step S17.

At the first step S11, a chitosan is prepared. An acid aqueous solution is added to the chitosan to dissolve the chitosan. As a result of dissolving the chitosan, a chitosan sol is produced. For example, the chitosan sol includes a chitin chitosan, a physiological saline solution, and an acid. As the acid, use can be made of malic acid, malonic acid, or the like.

At the second step S12, an apatite powder is added to or mixed with the chitosan sol. As the apatite powder, use is made of chemically synthesized which is made of hydroxiapatite of α-tricalcium phosphate and/or β-tricalcium phosphate.

At the third step S13, a mixture of the apatite powder and the chitosan sol is kneaded into a kneaded mixture.

At the fourth step S14, the kneaded mixture is neutralized by the aqueous solution including the compound. As a result of neutralization, the kneaded mixture has a pH value of 7.0-10.0. In this event, the compound includes at least one element selected from the monovalent and the divalent metal elements. In the example, as the monovalent metal compound include in the solution, use is made of at least one of sodium chloride, sodium bicarbonate and sodium polyphosphate. As the divalent metal compound include in the solution, use is made of at least one of zinc oxide and calucium silicate.

At the fifth step S15, a drying is carried out as regards the kneaded mixture for asepticization to produce a dried substance. At the sixth step S16, the dried substance is asepticized by ethylene oxide gas under high pressure. The dried substance is the material that is easy to break by adding external force.

At the seventh step S17, the dried substance is adsorbed with the Ringer's solution on or before use of the osteoconduction substance. As a result the dried substance is processed into an elastic body as the osteoconduction substance that has the elastic strength like rubber.

More particularly, the dried substance is dipped about 5-10 minutes into the Ringer's solution adjusted to have the pH value of 7.0-10.0. The osteoconduction substance caused the Ringer's solution absorbed becomes the elastic body like rubber. After that, the osteoconduction substance is cut into a plurality of film pieces each of which may have an optimal size in correspondence to a diseased part. For example, the film piece may be inserted between a jaw bone and a gingiva surrounding the jaw bone after operation for periodontitis in order to reconstruct the jaw bone and to prevent recurrence of periodontitis.

In the manner which will presently be described, comparison was carried out among the osteoconduction substances that are manufactured by using the Ringer's solution, physiological saline solution, and distilled water, respectively. In each of the following examples, it is to be noted that the chitosan sol includes physiological saline solution and, malic acid and, chitosan and that the osteoconduction substance is formed in a film of a thickness of 0.5mm and then cut to have a width of 2mm and a length of 3mm.

### [Example 1]

In example 1, the dried substance was processed into the osteoconduction substance by using the Ringer's solution. The osteoconduction substance will be called a first osteoconduction substance. In this event, dissolving 0.33g of CaCl₂, 0.3g of KCI, and 8.6 g of NaCl into the distilled water made the Ringer's solution of 1,000ml. In addition, the pH value of the osteoconduction substance was adjusted by sodium hydroxide and carbonic acid hydrogen sodium. The asepticization under the high pressure is carried out at 120°C during 20 minutes.

### [Comparative Example 2]

In comparative example 2, the dried substance was processed into the osteoconduction substance by using the distilled water. The osteoconduction substance will be called a second osteoconduction substance.

### [Comparative Example 3]

In comparative example 3, the dried substance was processed into the osteoconduction substance by using the physiological saline solution. The osteoconduction substance will be called a third osteoconduction substance.

Next, the description will be directed to animal tests by using the osteoconduction substances obtained in example 1, comparative example 2, and comparative example 3.

The periosteum of the head skeleton of each of four SD corallary rats was stripped. The osteoconduction substances were implanted in the rats to the head skeleton under the periosteum, respectively. After that, the rats ware slaughtered after one week, two weeks, three weeks, four weeks, and eight weeks respectively. Bone fragments of the rats were gathered that was crowded the osteoconduction substance. The bone fragments were soaked in formalin solutions of 10% so as not to putrefy. Next, decalcification was carried out as regards the bone fragments with 10% of ethylenediamine-tetraacetic acid-disodium solutions. After that, each of the bone fragments was wrapped by paraffin.

Various flaps of about 4 µm were produced from the bone fragments, respectively. The flaps were performed with hematoxylin eosin staining and then observed by an optical microscope in the manner known in the art. The result of the osteogenesis for each week is the following street.

### [Condition after 1 week]

In use of the first osteoconduction substance, the new bone was observed at both ends of the osteoconduction substance right under of the osteoconduction substance.

In use of the second osteoconduction substance, the new bone is hardly observed. The osteoconduction substance was wrapped by connective tissue. The distance between the osteoconduction substance and the bone of a living body are wide yet

In use of the third osteoconduction substance, the new bone was observed in the place where is near the both ends of only the osteoconduction substance. The connective tissue was admitted between the osteoconduction substance and the bone of the living body.

### [Condition after 2 weeks]

In use of the first osteoconduction substance, the new bone was growing at the both ends of the osteoconduction substance. It was observed that a part of the osteoconduction substance is substituted with the new bone right under the osteoconduction substance.

In use of the second osteoconduction substance, the connective tissue progresses and the osteoconduction substance is being absorbed barely. At the both ends of the osteoconduction substance, the formation of the new bone was barely seen.

In use of the third osteoconduction substance, the new bone of the both ends of the osteoconduction substance was growing. The osteoconduction substance is being absorbed barely.

### [Condition after 4 weeks]

In use the first osteoconduction substance, the osteoconduction substance is distributed fairly. The part that new bone is formed was observed a lot.

In use of the second osteoconduction substance, dispersion of the osteoconduction substance is advancing. Connective tissue does binary entrance and is progressing. It was observed that the new bone of the both ends of the osteoconduction substance is growing.

In use of the third osteoconduction substance, the new bone of the both ends of the osteoconduction substance was growing. The osteoconduction substance is distributing fairly and the connective tissue is being absorbed and is being do binary entrance.

### [Condition after 8 weeks]

In use of the first osteoconduction substance, the new bone of the both ends of the osteoconduction substance was further growing. About 1/2 of the osteoconduction substance were substituted with a new bone right under the osteoconduction substance. The osteoconduction substance in a near part distributes under the skin and absorption was advancing.

In use of the second osteoconduction substance, the osteoconduction substance is distributing fairly. The connective tissue is being absorbed and is being do binary entrance. The new bone was growing barely than 4 weeks.

In use of the third osteoconduction substance, the new bone of the both ends of the osteoconduction substance was growing. The osteoconduction substance is distributing fairly. However, is was not observed that the osteoconduction substance has a part connected with a new bone.

With the above-mentioned method, it becomes possible to provide the first osteoconduction substance which is capable of accelerating creation of the new bone. The osteoconduction substance of this invention is useful in dental, orthopedic, and plastic surgery fields as a filling or cushioning material for root canal filling, bone filling, a tooth support in pyorrhea alveolaris.

The third step S13 in Fig. 2 may be omitted from the method of manufacturing the osteoconduction substance.

## Claims

1. A method of manufacturing a substance suitable for use as an osteoconduction substance, comprising the steps of:
dissolving a chitosan by an acid aqueous solution to produce a chitosan sol;
mixing an apatite powder in said chitosan sol to produce a mixture;
adjusting the pH value of said mixture by sodium hydroxide and carbonic acid hydrogen sodium to a pH value of 7.0 - 10.0;
drying said mixture into a dried substance;
carrying out asepticization of said dried substance under high pressure after the drying step; and
adsorbing Ringer's solution in said dried substance to produce an elastic body as said substance.

2. The method as claimed in claim 1, further comprising the step of forming said mixture into a film shape after the mixing step.

3. A substance obtainable by the method according to claim 1.

4. The substance as claimed in claim 3, which is formed in a film shape.

5. An osteoconduction substance obtainable by the method according to claim 1 for use as a medicament.

6. Use of the osteoconduction substance obtainable by the method according to claim 1 for the preparation of a pharmaceutical composition for the acceleration of bone formation.

## Patentansprüche

1. Verfahren zur Herstellung eines Materials, das zur Verwendung als osteokonduktives Material geeignet ist, umfassend die Schritte:
das Auflösen von Chitosan in einer sauren wässrigen Lösung zur Herstellung eines Chitosan-Sols,
Mischen eines Apatit-Pulvers im Chitosan-Sol zur Herstellung eines Gemisches,
Einstellen des pH-Werts des Gemisches mit Natriumhydroxid und Natriumhydrogencarbonat auf einen pH-Wert von 7,0 bis 10,0,
Trocknen des Gemisches zu einem getrockneten Material,
Aseptisch-Machen des getrockneten Materials unter Hochdruck nach dem Trocknungsschritt und
Adsorbieren einer Ringer-Lösung in dem getrockneten Material zur Herstellung dieses Materials als elastischen Körper.

2. Verfahren gemäß Anspruch 1, das zusätzlich die Filmbildung des Gemisches nach dem Vermischungsschritt umfasst.

3. Material erhältlich nach dem Verfahren gemäß Anspruch 1.

4. Material gemäß Anspruch 3, das in Gestalt eines Films gebildet ist.

5. Osteokonduktives Material erhältlich nach dem Verfahren gemäß Anspruch 1 zur Verwendung als Arzneimittel.

6. Verwendung des nach dem Verfahren gemäß Anspruch 1 erhältlichen osteokonduktiven Materials für die Herstellung eines Arzneimittels zur Beschleunigung der Knochenbildung.

## Revendications

1. Procédé de fabrication d'une substance appropriée pour une utilisation en tant que substance avec une ostéoconduction, comprenant les étapes consistant à :
dissoudre un chitosan par une solution aqueuse d'acide pour produire un sol de chitosan ;
incorporer une poudre d'apatite au dit sol de chitosan pour produire un mélange ;
ajuster la valeur du pH dudit mélange par l'hydroxyde de sodium et par le sodium d'hydrogène de l'acide carbonique à une valeur de pH allant de 7,0 à 10,0 ;
sécher ledit mélange en une substance sèche ;
effectuer une aseptisation de ladite substance sèche sous une pression élevée après l'étape de séchage ; et
adsorber une solution de Ringer dans ladite solution sèche pour produire un corps élastique comme étant ladite substance.

2. Procédé comme revendiqué dans la revendication 1, comprenant, en outre, l'étape consistant à former ledit mélange selon une forme de film après l'étape de mélange.

3. Substance qui peut être obtenue par le procédé selon la revendication 1.

4. Substance comme revendiqué dans la revendication 3, qui est formée en une forme de film.

5. Substance avec une ostéoconduction qui peut être obtenue par le procédé selon la revendication 1 pour une utilisation comme médicament.

6. Utilisation de la substance avec une ostéoconduction qui peut être obtenue par le procédé selon la revendication 1 pour la préparation d'une composition pharmaceutique pour l'accélération d'une formation osseuse.
